# EUROPEAN PATENT SPECIFICATION

(11) **EP 2 558 038 B1**
(45) Date of publication and mention of the grant of the patent: **30.09.2015**
(21) Application number: 11716628.0
(22) Date of filing: 12.04.2011
(51) Int. Cl.: A61F 2/78

(54) **FAIL-SAFE ATTACHMENT FOR PROSTHETIC LIMB**
AUSFALLSICHERE HALTERUNG FÜR GLIEDMASSENPROTHESE
FIXATION À SÉCURITÉ INTRINSÈQUE POUR PROTHÈSE DE MEMBRE

(30) Priority: 12.04.2010 GB 201006047
(43) Date of publication of application: 20.02.2013
(73) Proprietor: Stanmore Implants Worldwide Limited, Elstree, Hertfordshire WD6 3SJ (GB)
(72) Inventor: WOZENCROFT, Robert Michael, Epsom, Surrey KT19 8SS (GB); ORME, Katrina, Elstree, Hertfordshire WD6 3SJ (GB)
(74) Representative: Maughan, Sophie Louise
(86) International application number: PCT/GB2011/050716
(87) International publication number: WO 2011/128674

(56) References cited:
- WO-A2-2007/018904

## Description

This invention relates to a fail-safe attachment for a prosthetic limb, such as an exoskeletal prosthesis.

In the case where limbs are amputated, it is often necessary to provide the patient with an exoskeletal prosthesis. These prostheses allow the user to maintain an active life, but there is a need to safeguard against further damage being done should the prosthesis be subject to an overload force, for example during a fall or other unexpected incident. During such situations, it is better that the prosthesis moves or even becomes detached, rather than transferring excessive forces to the residual limb. For this reason, some prostheses can be attached using fail-safe mechanisms, examples being such as GB patent 2411841B or US patent application 2008/0288087 , which is considered to represent the closest prior art and discloses all the features of the preamble of claim 1. The present invention attempts to provide an improvement to this type of attachment.

Accordingly, a fail-safe attachment is provided for coupling an exoskeletal prosthesis to a patient, said fail-safe attachment comprising:
a first component adapted for connection to the patient; and
a second component adapted for connection to the prosthesis;
the fail-safe attachment further including a safety release mechanism normally retaining the prosthesis in a substantially motion-free interconnected relation to the patient, said safety release mechanism responding to a force overload condition to permit relative movement between the prosthesis and the patient;
said safety release mechanism comprising a bending force release mechanism responsive to a bending force overload condition to permit the separation of said second component relative to said first component, the bending force release mechanism comprising a plurality of spring loaded jaws located on a first one of either the first and second components, and being adapted to engage a complementary portion of a second one of either the first and second components, the jaws being pivotable in an over-centre spring configuration between a first position in which they engage the complementary portion to secure the first and second components one to the other, and a second position in which the first and second components are able to separate.

Typically, a limb of the patient has a bone anchored mounting post with an external fixation structure, and the first component is adapted for connection to the fixation structure. Conveniently, the safety release mechanism of the fail-safe attachment normally retains the prosthesis in a rigid substantially motion-free interconnected relation to the fixation structure.

The bending force release mechanism is responsive to a bending force overload, which is a severe force attempting to displace the second component relative to the first component such that their longitudinal axes are no longer coaxial one with the other. This is typically encountered when the prosthesis is subjected to an angular force, attempting to change the longitudinal axis of the prosthesis, for example by means of a sideways force applied to one end of the prosthesis. The bending force release mechanism is responsive to any off-axis force, or combination of forces, other than a circumferential rotational force. The bending force release mechanism, with its over-centre configuration of the jaws, provides a reliable and effective mechanism for attaching the exoskeletal prosthesis to a connecting component, while allowing for efficient detachment in the event of an overload force. Conveniently, the spring loaded jaws each comprise a radially extending arm having an inner end an outer end, the outer end of each arm portion carrying a finger portion. In a convenient arrangement the finger portions extend in retrograde fashion with respect to each arm, while in an alternative arrangement the finger portions conceivably extend outwardly with respect to each arm. The inner end of each arm is preferably attached to a piston movable between a first position in which the inner end of each arm is lower than the outer end, and a second position in which the inner end of each arm is higher than the outer end. Each arm is preferably provided with a compression spring, such that the compression spring is compressed to a greater extent when the arms are at any position between their first and second positions, and to a lesser extent when the arms are at their first or second positions, thereby to bias the arms into their first and second positions. In this way, the over-centre mechanism provides two stable positions for the arms, a first in which the jaws engage the complementary portion, and a second in which the components are free to become separated one from the other. The arms are biased by the compression springs into either their first and second positions, and require a predetermined force to overcome this biasing in order to move between the two positions. In this way, the jaws will require a predetermined force before they are able to be moved from their "closed" position into their "open" position. Typically, the piston is movable on a spindle.

Conveniently, the complementary portion engaged by the spring loaded jaws comprises a flange. Typically, the flange is present on the first component, and the spring loaded jaws are present on the second component. In this way, the first component is attached to the user's limb, and the second component to the prosthesis. When in normal use, the spring-loaded jaws engage the flange and secure the prosthesis in place.

The spring loading for the jaws is preferably assisted by a load cell unit capable of exerting a preset force on the bending force release mechanism. Where a piston is provided as described above, the load cell unit conveniently acts on the piston to urge it into its first position. Any bending overload force has to overcome the preset force exerted by the load cell unit on the piston, as well as the force exerted by the compression springs on the arms, before the piston moves to relax the jaws and release the first component from the second component. Thus the load cell unit sets the level of the bending overload force necessary to activate the fail-safe mechanism.

A mechanical reset mechanism is conveniently provided in order to move the jaws between their first and second positions. The mechanical reset mechanism typically comprises a lever acting on the piston to move it between its first and second positions. In this way, the user is able to manually move the jaws between their open and closed positions, for example to re-attach the prosthesis after the fail-safe mechanism has caused it to become detached. Alternatively, the user may simply push the first and second components together, snapping the jaws back into their closed position, without using the mechanical reset mechanism, or manually press down on the load cell, pressing down the piston and setting the jaws back into their engaged position.

Typically, the safety release mechanism further comprises a torsion fail-safe mechanism responsive to a torsion force overload condition to permit relative rotational displacement of said second component relative to said first component. The torsion fail-safe mechanism is responsive to a torsion force overload, which is a severe rotational force applied to the second component relative to the first component, the rotational force being directed circumferentially around the longitudinal axis of the second component. This is typically encountered when the prosthesis is subjected to a twisting force, attempting to rotate the prosthesis circumferentially around its longitudinal axis. Whichever type of overload force or combination of forces (i.e. bending and/or torsion forces) is encountered, the safety release mechanism is capable of dealing with the force so as to prevent damage to the user.

Preferably the torsion fail-safe mechanism is a torsion force clutch responsive to a torsion force overload condition to permit relative rotational displacement of said second component relative to said first component. The torsion force clutch preferably comprises a clutch plate acting on a flange to restrict relative rotational movement between the first and second components. This provides the fail-safe mechanism with its operation when faced with a torsional or rotational force. The jaws conveniently act on the clutch plate to urge it against the flange. It is only when the torsional overload force exceeds a preset level that the clutch plate allows rotation of the first and second components relative to one another. In this way, when subjected to an excessive rotational force, the prosthesis will rotate with respect to the remainder of the attachment rather than transmitting the rotational force to the limb of the user. As before, a mechanical reset mechanism is conveniently provided in order to release the pressure exerted by the clutch plate in order to reset the orientation of the first and second components relative to each other. Following activation of the rotational fail-safe mechanism, the user may operate the mechanical reset mechanism to allow the prosthesis to be re-oriented back to its original position. This can be achieved by rotating the first and second components relative to one another, i.e. without separating them one from another. Alternatively, the first and second components can be detached one from the other, the clutch plate rotated back to its original position, and the first and second components re-connected one to the other in the desired orientation. Conveniently, a single mechanical reset mechanism is provided in order to reset both the torsion force clutch and the bending force release mechanism.

As before, the spring loading for the jaws is preferably provided by the combination of the compression springs on the arms, and by a load cell unit capable of exerting a preset force on the clutch plate and hence the torsion force clutch. Conveniently, a single load cell unit contributes to the preset force for both the torsion force clutch and the bending force release mechanism. The load cell unit typically includes a compression spring adapted to contribute to the preset force for both the torsion force clutch and/or the bending force release mechanism. A choice of load cell units is preferably provided, each load cell unit offering a different preset force for the torsion force clutch and the bending force release mechanism. The load cell unit may include one or more compression springs, each spring in the load cell contributing to the preset force for the torsion force clutch and/or the bending force release mechanism. This allows for various different levels of fail-safe mechanism to be employed, ranging from the sensitive (in which only a relatively small bending force or torsional force is required to activate the fail-safe mechanism) to the robust (in which a much larger bending force or torsional force is required to activate the fail-safe mechanism). Conceivably, the most sensitive setting is with no load cell present, i.e. with the preset force for both the torsion force clutch and the bending force release mechanism being provided solely by the spring loading for the jaws.

The invention further resides in a fail-safe attachment for coupling an exoskeletal prosthesis to a patient limb having a bone anchored mounting post with an external fixation structure, said fail-safe attachment comprising:
a first component adapted for connection to the fixation structure; and
a second component adapted for connection to the prosthesis;
the fail-safe attachment further including a safety release mechanism normally retaining the prosthesis in a rigid substantially motion-free interconnected relation to the fixation structure, said safety release mechanism responding to a force overload condition to permit relative movement between the prosthesis and the fixation structure;
said safety release mechanism comprising a torsion force clutch responsive to a torsion force overload condition to permit relative rotational displacement of said second component relative to said first component, and a bending force release mechanism responsive to a bending force overload condition to permit the separation of said second component relative to said first component; and
a biasing means capable of exerting a preset force on both the torsion force clutch and the bending force release mechanism so as to establish the torsion overload force and the bending overload force necessary to operate each of the torsion force clutch and the bending force release mechanism respectively.

Conveniently, the biasing means comprises a load cell unit, typically able to regulate both the bending overload force necessary to activate the fail-safe mechanism, but also the torsional overload force necessary to activate the fail-safe mechanism. Preferably, the load cell unit includes a compression spring adapted to contribute to the preset force for both the torsion force clutch and the bending force release mechanism. As before, a choice of load cell units is conveniently provided, each load cell unit offering a different preset force for the torsion force clutch and the bending force release mechanism.

The invention will now be described in more detail, by way of example only, with reference to the accompanying drawings, in which;
Figure 1 is a perspective view of a fail-safe attachment in accordance with the present invention;
Figure 2 is a exploded perspective view of the fail-safe attachment of Figure 1;
Figure 3 is a exploded sectional view of the fail-safe attachment of Figure 1;
Figure 4 is a sectional view of the attachment of Figure 1, shown in its assembled configuration;
Figure 5 is an exploded view of the top assembly of Figure 1, showing all of the various components;
Figure 6 is a schematic cross-sectional view of the top assembly of Figure 5;
Figure 7 is an enlarged view of a section of Figure 6, showing the cam mechanism in more detail;
Figure 8 is an exploded view of the base assembly of Figure 1, showing all of the various components;
Figure 9 is an exploded view of one of the jaw assemblies of Figure 8, showing it in more detail;

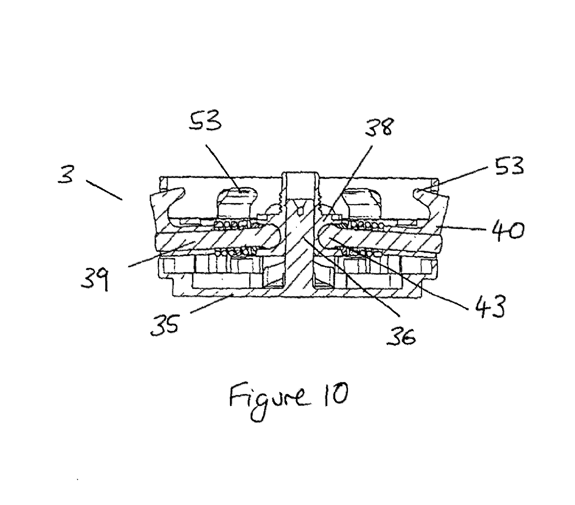

Figure 10 is a cross-sectional view of the base assembly of Figure 8, shown with the jaws in their open configuration; and
Figure 11 is an exploded view of the load cell of Figures 2 to 4.

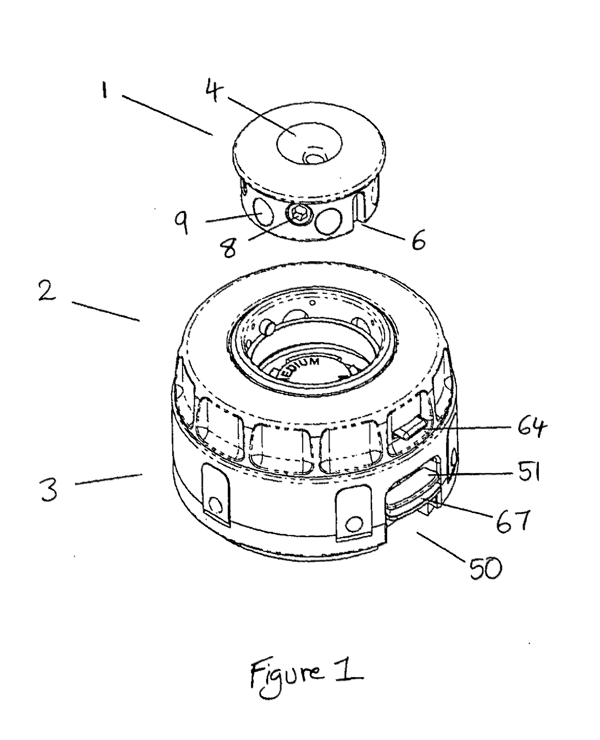

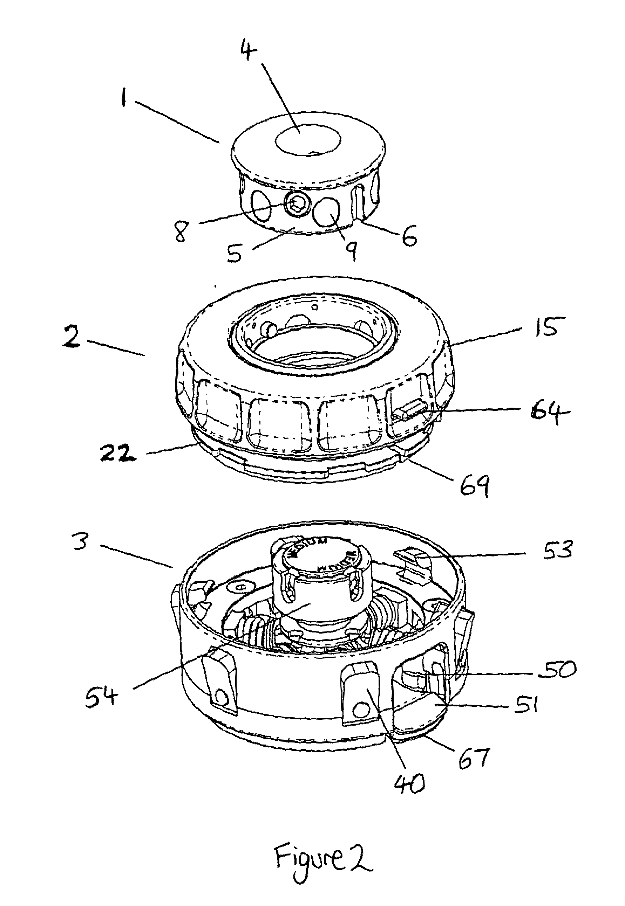

Referring to Figures 1 to 4, a fail-safe attachment comprises an external fixation structure in the form of an end cap 1, a first component in the form of a top assembly 2 and a second component in the form of a base assembly 3, all connectable one to another and all to be described in more detail subsequently. The end cap 1 has a generally cylindrical shape, and includes a central recess 4 designed to receive the shaft (not shown) of a transcutaneous implant emerging from the body of a patient. The base assembly 3 is connected to (or forms part of) a prosthesis, typically a prosthetic leg or other limb. The attachment system is the means by which the prosthetic limb is attached to the patient.

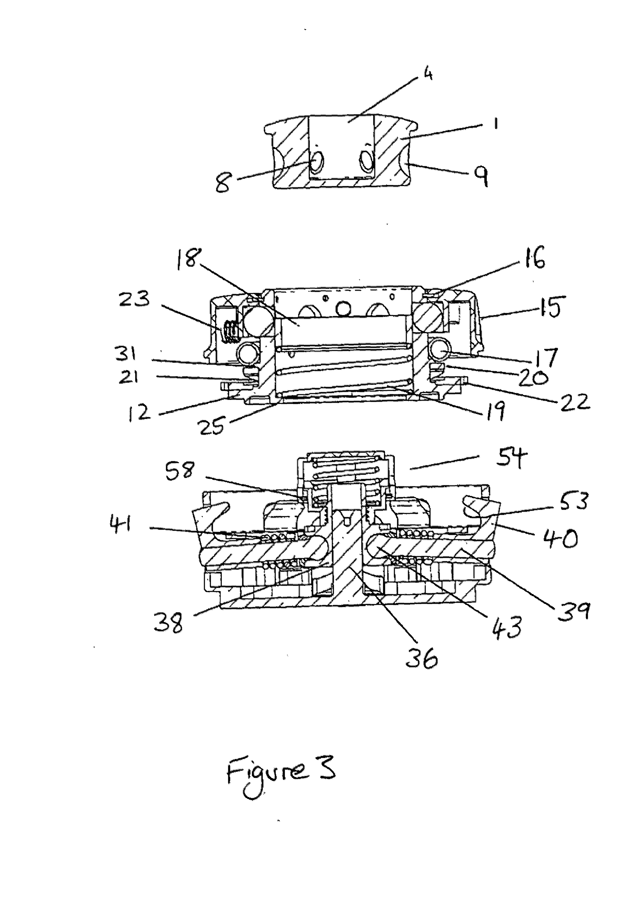

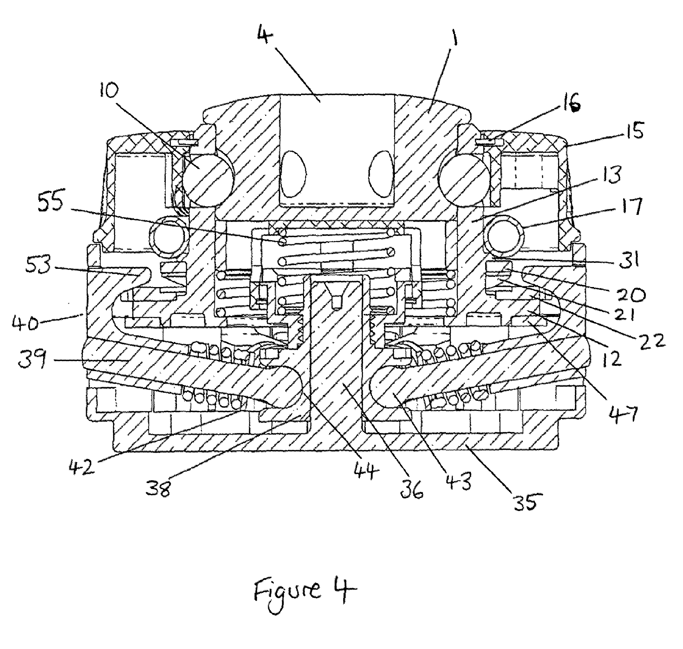

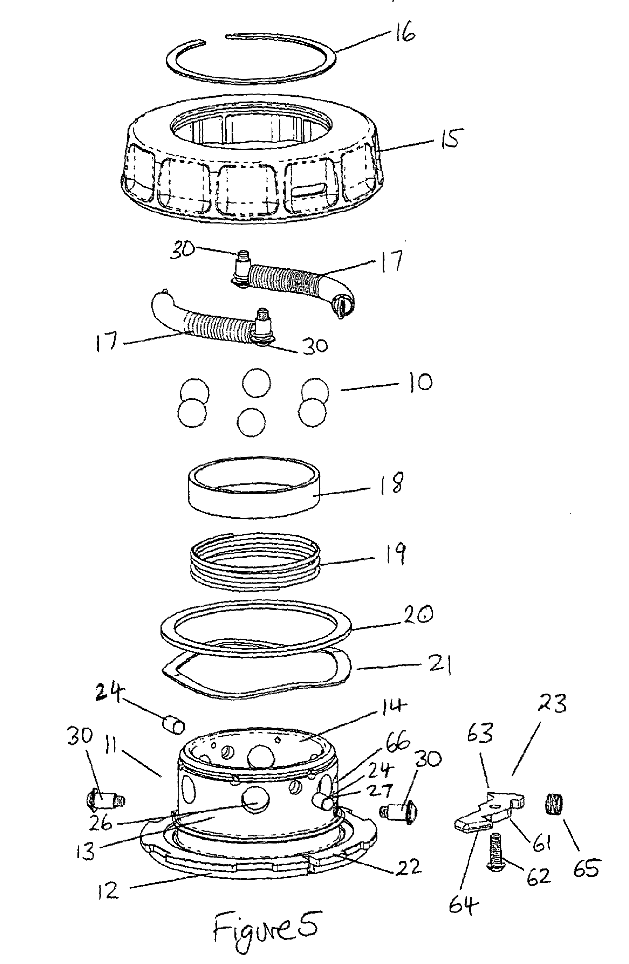

The end cap 1 has a cylindrical side wall 5, which is provided with slots 6, retaining screws 8, and a plurality of seats 9 adapted to receive ball bearings 10 forming part of the top assembly 2. The top assembly 2 includes a housing 11, comprising a flange 12 and a cylindrical body 13, the cylindrical body 13 having a central bore 14 such that the end cap 1 can be received therein. The top assembly includes a number of components, shown in exploded fashion in Figure 5, and listed below. These comprise release dial 15, first retaining ring 16, extension springs 17, ball collar 18, compression spring 19, second retaining ring 20, wave spring 21, clutch plate 22, interlock mechanism 23 and anti-rotation pins 24. The operation of these components will now be described, with particular reference to Figures 3 and 4.

Compression spring 19 is received within the bore 14 of the cylindrical body 13, and sits against an internal shoulder 25 at the bottom of the bore. The ball collar 18 sits above the compression spring 19, such that it can be pushed downwards against the action of the compression spring when the end cap 1 is received in the bore 14. Release dial 15 is located over the body 13, and held in place by first retaining ring 16. Extension springs 17 sit underneath the release dial, and are held in place by pins 30 attached to the cylindrical body 13. The cylindrical body 13 also has a series of holes 26 for receiving the ball bearings 10, and two smaller holes 27 for receiving the anti-rotation pins 24.

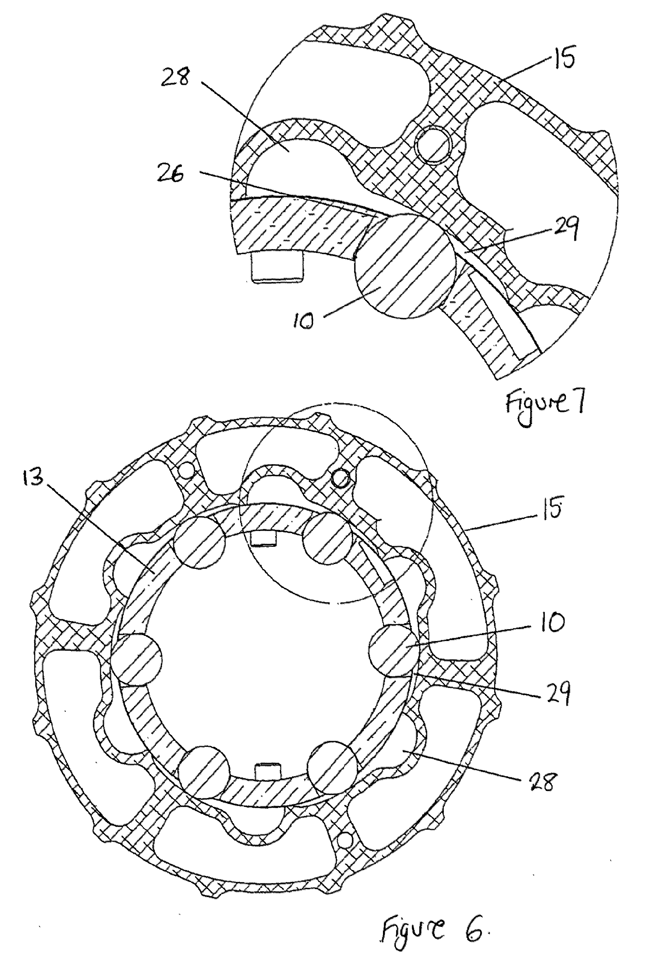

The release dial 15 has two rotational positions, a locked position and an unlocked position. The internal profile of the release dial, shown in Figures 6 and 7, includes a cam profile having first sections 28 and second sections 29. When the release dial is in its unlocked position, the ball bearings 10 are located wholly within the release dial in the first sections 28. Conversely, when the release dial is in its locked position, the ball bearings are located in the second sections 29 and protrude through the holes 26. With no end cap 1 present in the bore 14 of the housing 11, the ball collar 18 holds release dial in the unlocked position, with the ball bearings 10 being held within the release dial 15.

To connect the end cap 1 to the housing 11, the end cap is presented to the housing with the anti-rotation pins 24 aligned with the slots 6 so that the end cap 1 can be received within the bore 14. When the end cap 1 enters the bore 14, it pushes down the ball collar 18 against the action of the compression spring 19 exposing the holes 26 and the ball bearings 10 contained therein. When the end cap 1 is fully inserted, the ball bearings 10 are received in the seats 9 in the end cap. The release dial 15 then rotates into its locked position, under the action of the extension springs 17 thereby locking the end cap 1 firmly within the top assembly 2. The interlock mechanism 23 comprises a lever 61 pivotable about a pin 62 and including a protruding nose 63 at one end and a push button 64 at the other. When the release dial is in its locked position, the lever 61 of the interlock mechanism 23 is urged, under the action of a spring 65, such that the nose 63 engages with a recess 66 provided on the cylindrical body 13. This holds the release dial 15 in its locked position and prevents unintentional rotation of the dial.

When the user wishes to release the prosthesis, the user presses the button 64 to withdraw the nose of the interlock mechanism from the recess 66, and rotates the release dial 15 into its unlocked position against the action of the extension springs 17. This causes the ball bearings to retract into the release dial using the first sections 28 of the cam profile. The end cap 1 can then be removed from the top component 2, thereby removing the prosthesis. As the end cap 1 is removed, the ball collar 18 rises under the action of the compression spring 19 to hold the ball bearings 10 within the release dial 15 to maintain the release dial in its unlocked position, ready for the end cap to be reinserted when required. In this way, the release dial is moved between its locked position in which the end cap 1 is held firmly within the bore, and its unlocked position, in which the end cap 1 can be inserted or withdrawn from the bore 14. The user is therefore able to attach and detach the prosthesis easily and effectively.

The clutch plate 22 sits on the flange 12 and is held in place by means of the wave spring 21 and second retaining ring 20. The second retaining ring 20 fits under an upper flange 31 present on the outside of the cylindrical body 13.

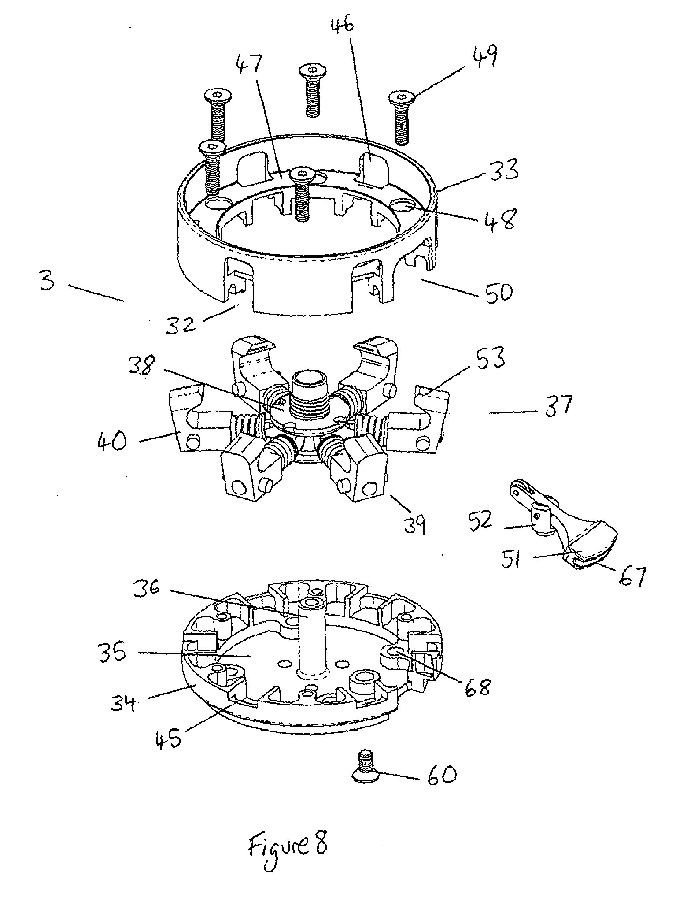

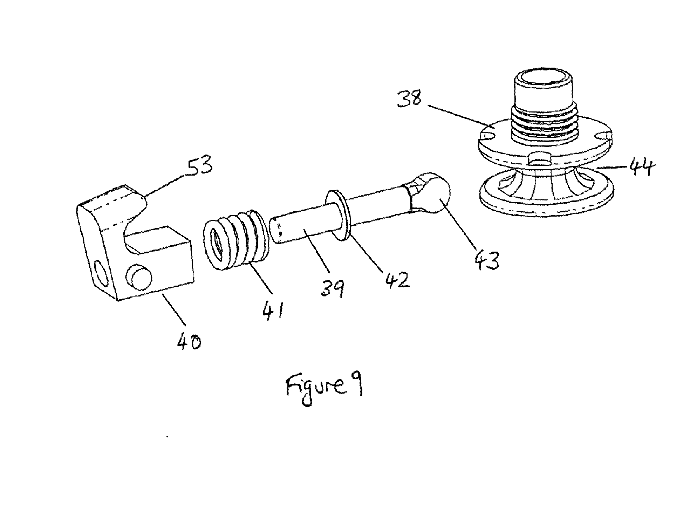

The base assembly 3 includes a housing comprising an upper part 33 and a lower part 34, as shown in Figures 8 and 9. The lower part 34 is in the form of a cup with a base 35 and a central spindle 36. Located on the spindle 36 is a jaw assembly 37, comprising a piston 38, and arms 39, each arm having a jaw member 40 at the end thereof. Each arm is also provided with a spring 41 and a washer 42, providing tension between the jaw members 40 and the central piston 38. Each arm 39 has a rounded proximal portion 43, located within a groove 44 present on the piston 38. The jaw members 40 extend into cut-outs 45 present around the circumference of the lower part 34. The cut-outs 45 cooperate with complementary cut-outs 46 present in the upper part 33 so as to form apertures 32 into which the jaw members extend. The jaw members 40 are provided with fingers 53, extending in a retrograde manner with respect to the arms 39.

The upper part 33 is also cup-shaped and includes a flange 47 with holes 48, through which screws 49 are used to secure the upper and lower parts one to the other. The upper and lower parts also include one additional larger cut-out so that they form an additional aperture 50 through which a release lever 51 is able to extend. The release lever 51 is secured in place by screw 60, and is movable about a fulcrum 52 to act on the piston 38. The far end of the release lever 51 fits into the groove 44 such that movement of the lever can urge the piston up or down on the spindle 36. The exposed end of the release lever 51 includes a slot 67, enabling the lever to be operated by a key or a coin (not shown).

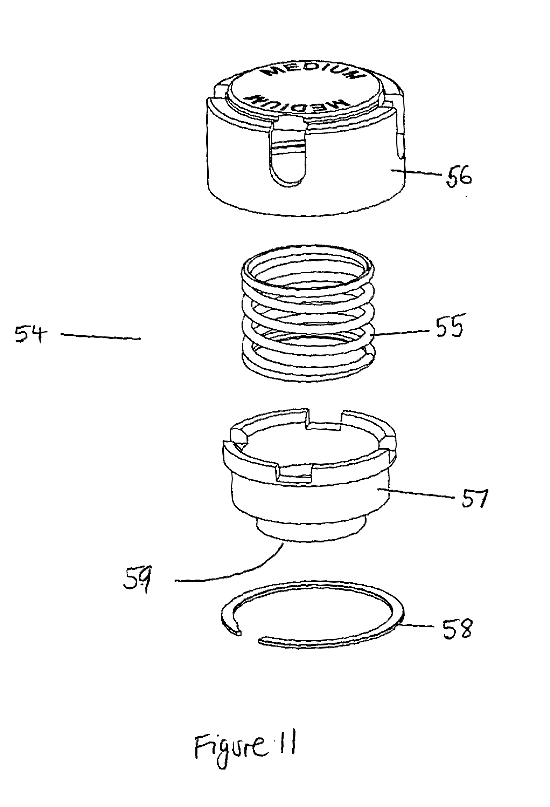

The final component of the attachment system is a pre-load cell 54, shown in Figure 11. This component comprises a compression spring 55, contained in the enclosure between upper and lower housings 56 and 57, and held one against the other by a retaining ring 58. The lower housing 57 has a central aperture 59, such that the pre-load cell 54 can be located over the spindle 36 to sit on top of the piston 38. This pre-load cell 54 is used to set the forces needed to operate the fail-safe features of the attachment mechanism. A plurality of pre-load cells can be provided, each with a different grade of compression spring, to produce different force levels at which the fail-safe features operate (as will be described further in greater detail).

The base assembly 3 is attached to a prosthesis (such as a prosthetic leg) by means of screws (not shown) using screw holes 68. The top assembly 2 is located into the base assembly 3, with pre-load cell 54 in position on the spindle 36. The jaw assembly 37 operates as an "over-centre" mechanism, as will be further described. The piston 38 is movable on the spindle 36 between two equally stable positions, one either side of the centre line in which the arms are horizontal. The springs 41 are compressed less when the arms 39 are in their two equally stable positions, as compared with any point in between. In the "open" position, the piston 38 is raised on the spindle 36, with the arms 39 pointing downwardly as extending away from the piston, such that the jaw members 40 are tilted outwardly in the apertures 46. The top assembly 2 is pushed down into the base assembly 3, and the load cell 54 is manually depressed to move the piston 38 downwardly on the spindle 36. Alternatively, the lever 51 can be operated to move the piston 38 downwardly on the spindle 36. Either way, the jaw assembly is moved into its "closed" position. In this position, the arms 39 point upwardly as extending away from the piston, such that the jaw members 40 are upright such that the fingers 53 can engage the clutch plate 22 of the top assembly 2 and hold it securely within the base assembly.

In use the end cap 1 is inserted into the top assembly 2, and locked in position as previously described. In this way, the prosthetic leg attached to the base assembly is connected to the user. In normal use, the prosthesis is attached and detached in this way, but the operation of the attachment system under abnormal conditions will now be described.

The attachment assembly provides a fail-safe mechanism by which the prosthesis is held rigidly under normal conditions of use, but is allowed to rotate or become detached if subjected to forces in excess of a predetermined threshold. Firstly, the operation of the attachment mechanism will be described when the prosthesis is subjected to a torsional (rotational) overload force. In normal operation, rotational movement between the top assembly 2 and base assembly 3 is resisted by the clutch plate 22 pressing the top assembly flange 12 into frictional engagement against the base assembly flange 47 (as shown in Figure 4). This is achieved by the fingers 53 pressing the clutch plate 22 against the flange 12 under the action of the springs 41 in the arms 39 and the compression spring 55 in the pre-load cell 54. However, when the torsion overload force exceeds a predetermined level, the flange 12 will rotate with respect to the clutch plate and the flange 47, thereby allowing the base assembly 3 to rotate with respect to the top assembly 2. This will allow the prosthesis to rotate rather than risk injury to the user by transferring an excessive rotational force to the remaining bone.

The result of this movement will be that the prosthesis becomes rotationally misaligned, but this can be reset using the key and the release lever 51. Operation of the release lever 51 moves the piston 38 upwardly on the spindle 36, moving the jaw assembly 37 to its "open" position and relieving the pressure of the fingers 53 against the clutch plate 22. This allows the base assembly 3 to be easily rotated with respect to the top assembly 2, back into its desired rotational orientation. Alternatively, once the release lever 51 has been operated to move the jaw assembly 37 into its "open" position, the top assembly 2 and base assembly 3 can be separated one from the other, and reconnected when they have been rotated into the desired orientation. A slot 69 present on the clutch plate 22 (and also sized such as to receive the key or a coin) allows the user to orient the base assembly into its original position. Whichever way the rotational orientation is achieved, once the base assembly 3 is in its desired position, the top and bottom assemblies are secured in position one to the other. This can be achieved by operating the release lever 51 to move the piston 38 back into its "closed" position, thereby locking the top and bottom assemblies in place once again. Alternatively, the user can manually press down on the pre-load cell 54, snapping the jaw assemblies 37 back into their "closed" position.

The force at which the base assembly 3 will rotate relative to the top assembly 2 will depend on the force exerted by the springs 41 in the arms 39 and by the compression spring 55 in the pre-load cell 54. Providing different pre-load cells (typically colour-coded) with different grades of compression spring, allows for different degrees of fail-safe operation. A relatively low force compression spring will mean that the fail-safe mechanism operated relatively easily and allows for rotation at relatively low torsional forces. In contrast, a different pre-load cell with a higher force compression spring will mean that the fail-safe mechanism only allows rotation at a relatively higher level of torsional force. The provision of different pre-load cells allows the same design of attachment mechanism to be employed for all types of users.

The operation of the attachment mechanism will now be described when the prosthesis is subjected to a bending overload force, typically experienced when the prosthesis is subjected to an unexpected off-axis or lateral force. Normally the top assembly 2 is prevented from becoming detached from the base assembly 3 by means of the jaw members 40, which are in their upright position such that the fingers 53 hold the top assembly flange 12 against the base assembly flange 47. This is the case when the piston 38 is in its lower position on the spindle 36, and the arms 39 are in the upwardly pointing position of the over-centre mechanism described previously. The piston 38 and arms 39 are maintained in this "closed" position by the action of the springs 41 in the arms 39, and by the compression spring 55 in the pre-load cell 54 acting on the piston 38 to keep it in the downwards position.

However, when a bending force is experienced by the prosthesis, this causes the flange 47 of the base assembly 3 to move away from the flange 12 of the top assembly 2 in at least one circumferential region, and to hinge against the flange 12 in another circumferential region. In doing so, the flange 12 of the top assembly 2 pushes against the jaw assembly 37 in a circumferential region. When the bending forces exceed a predetermined threshold, they overcome the force exerted by the springs 41 and 55, and the piston 38 moves upwardly on the spindle 36. This allows the arms 39 to move to their "open" position, causing the fingers 53 to snap back to their outwardly tilted position and allow the flange 12 to be released. This causes the base assembly 3 and top assembly 2 to become detached one from the other, allowing the prosthesis to become disconnected from the user rather than transmitting an excessive force to the remaining bone.

As before, the force at which this detachment occurs is dependent on the combination of the springs 41 in the arms 39, and the compression spring 55 in the pre-load cell 54, and can hence be varied by deploying different pre-load cells having different strength compression springs. It will be noted that the compression spring of the pre-load cell governs both the threshold at which the rotational fail-safe operates and also the threshold at which the bending force fail-safe operates. It is therefore possible to easily and effectively adjust both thresholds simultaneously, by substituting a different pre-load cell 54. The pre-load cells may each contain one or more compression springs. By including more than one compression spring in a pre-load cell 54, this increases the threshold force necessary to activate the fail-safe mechanism.

It is possible to utilize the fail-safe attachment without the pre-load cell 54 being present. In this instance, the force at which detachment or rotation occurs is governed solely by the radial springs 41 in the arms 39. This constitutes the most sensitive setting for the fail-safe mechanism, as the force necessary for detachment or rotation is the lowest.

Figure 9 shows one washer 42 on each arm 39, however of course, more than one washer can be provided on each arm 39, thus providing a greater compressive force on spring 41.

It will of course be understood that torsion fail-safe mechanisms other than the particular torsion force clutch shown in the figures can be used with the bending force release mechanism described herein. For example, if the fail-safe attachment is provided without a clutch plate 22 and with a thicker flange 12, the attachment will provide only a bending force release mechanism and there will be no torsion fail-safe mechanism provided by the spring loaded jaws. If desired, an anti-rotation feature can be included to prevent the top assembly 2 and base assembly 3 rotating relative to one another, such as an anti-rotation pin or the like, or recesses in the top assembly 2 that the fingers 53 engage in to prevent relative rotation. A torsion fail-safe mechanism separate and independent from the bending force release mechanism could of course be provided. For example, the fail-safe attachment may have a torsion force clutch between the bending force release mechanism and the exoprosthesis. Alternatively, the fail-safe attachment may have no torsion fail-safe mechanism, but only a bending force release mechanism.

## Claims

1. A fail-safe attachment for coupling an exoskeletal prosthesis to a patient:
a first component (2) adapted for connection to the patient structure; and
a second component (3) adapted for connection to the prosthesis;
the fail-safe attachment further including a safety release mechanism normally retaining the prosthesis in a substantially motion-free interconnected relation to the patient, said safety release mechanism responding to a force overload condition to permit relative movement between the prosthesis and the patient;
said safety release mechanism comprising a bending force release mechanism responsive to a bending force overload condition to permit the separation of said second component relative to said first component, the bending force release mechanism comprising a plurality of spring loaded jaws (40) located on a first one of either the first and second components, and being adapted to engage a complementary portion of a second one of either the first and second components,
**characterised in that** the jaws (40) being pivotable in an over-centre spring configuration between a first position in which they engage the complementary portion to secure the first and second components one to the other, and a second position in which the first and second components are able to separate.

2. A fail-safe attachment according to claim 1, wherein the spring loaded jaws (40) each comprise a radially extending arm (39) having an inner end and an outer end, the outer end of each arm carrying a finger portion (53).

3. A fail-safe attachment according to claim 2, wherein the finger portion (53) of each arm extends in a retrograde fashion with respect to the arm (39).

4. A fail-safe attachment according to claim 2 or claim 3, wherein the inner end of each arm is attached to a piston (38) movable between a first position in which the inner end of each arm is lower than the outer end, and a second position in which the inner end of each arm is higher than the outer end.

5. A releasable attachment according to claim 4, wherein each arm is provided with a compression spring (41), such that the compression spring is compressed to a greater extent when the arms are at any position between their first and second positions, and to a lesser extent when the arms are at their first or second positions, thereby to bias the arms into their first and second positions.

6. A fail-safe attachment according to claim 4 or claim 5, wherein the piston (38) is movable on a spindle (36).

7. A fail-safe attachment according to any of claims 1 to 6, wherein the complementary portion engaged by the spring loaded jaws (40) comprises a flange (12).

8. A fail-safe attachment according to claim 7, wherein the flange (12) is present on the first component (2), and the spring loaded jaws (40) are present on the second component (3).

9. A fail-safe attachment according to any preceding claim, wherein the spring loading for the jaws (40) is assisted by a load cell unit (54) capable of exerting a preset force on the bending force release mechanism.

10. A fail-safe attachment according to any preceding claim, wherein a mechanical reset mechanism is provided in order to move the jaws (40) between their first and second positions.

11. A fail-safe attachment according to claims 4 and 10, wherein the mechanical reset mechanism comprises a lever (51) acting on the piston (38) to move it between its first and second positions.

12. A fail-safe attachment according to any preceding claim, wherein the safety release mechanism further comprises a torsion fail-safe mechanism responsive to a torsion force overload condition to permit relative rotational displacement of said second component relative to said first component.

13. A fail-safe attachment according to any preceding claim, wherein the safety release mechanism further comprises a torsion force clutch responsive to a torsion force overload condition to permit relative rotational displacement of said second component relative to said first component.

14. A fail-safe attachment according to claim 13, wherein the torsion force clutch comprises a clutch plate (22) acting on a flange (12) to restrict relative rotational movement between the first and second components.

15. A fail-safe attachment according to claim 14, wherein the jaws (40) act on the clutch plate (22) to urge it against the flange (12).

16. A fail-safe attachment according to any preceding claim, wherein a limb of the patient has a bone anchored mounting post with an external fixation structure (1).

17. A fail-safe attachment according to claim 16, wherein the first component is adapted for connection to the fixation structure, and the fail-safe attachment further includes a safety release mechanism normally retaining the prosthesis in a rigid substantially motion-free interconnected relation to the fixation structure.

## Patentansprüche

1. Eine ausfallsichere Halterung zur Verbindung einer exo-skeletalen Prothese mit einem Patienten, mit:
einer ersten Komponente (2), die zur Verbindung mit der Patienten-Struktur ausgebildet ist; und
einer zweiten Komponente (3), die zur Verbindung mit der Prothese ausgebildet ist;
wobei die ausfallsichere Halterung weiterhin einen Sicherheits-Auslösemechanismus einschließt, der die Prothese normalerweise in einer im wesentlichen bewegungsfrei verbundenen Beziehung zu dem Patienten hält, wobei der Sicherheits-Auslöse-Mechanismus auf einen Kraft-Überlast-Zustand anspricht, um eine Relativbewegung zwischen der Prothese und dem Patienten zu ermöglichen;
wobei der Sicherheits-Auslöse-Mechanismus einen Biegekraft-Auslösemechanismus umfasst, der auf einen Biegekraft-Überlast-Zustand anspricht, um die Trennung der zweiten Komponente gegenüber der ersten Komponente zu ermöglichen, wobei der Biegekraft-Auslösemechanismus eine Anzahl von federvorbelasteten Greifbacken (40) aufweist, die auf einer ersten von entweder der ersten und der zweiten Komponente angeordnet sind und die zum Eingriff mit einem komplementären Teil einer zweiten von entweder der ersten und zweiten Komponenten angeordnet sind,
**dadurch gekennzeichnet, dass** die Greifbacken (40) in einer Sprungwerk-Federkonfiguration zwischen einer ersten Position, in der sie mit dem komplementären Teil in Eingriff stehen, um die ersten und zweiten Komponenten aneinander zu befestigen, und einer zweiten Position verschwenkbar sind, in der sich die ersten und zweiten Komponenten trennen können.

2. Eine ausfallsichere Halterung nach Anspruch 1, bei der die federvorbelasteten Greifbacken (40) jeweils einen sich in Radialrichtung erstreckenden Arm (39) mit einem inneren Ende und einem äußeren Ende umfassen, wobei das äußere Ende jedes Armes einen Finger-Abschnitt (53) trägt.

3. Eine ausfallsichere Halterung nach Anspruch 2, bei der der Finger-Abschnitt (53) jedes Arms sich in einer retrograden Weise bezüglich des Arms (39) erstreckt.

4. Eine ausfallsichere Halterung nach Anspruch 2 oder Anspruch 3, bei der das innere Ende jedes Arms an einem Kolben (38) angebracht ist, der zwischen einer ersten Position, in der das innere Ende jedes Arms niedriger ist, als das äußere Ende, und einer zweiten Postion beweglich ist, in der das innere Ende jedes Arms höher als das äußere Ende ist.

5. Eine lösbare Halterung nach Anspruch 4, bei der jeder Arm mit einer Kompressionsfeder (41) derart versehen ist, dass die Kompressionsfeder in einem größeren Ausmaß komprimiert ist, wenn sich die Arme an irgendeiner Position zwischen ihren ersten und zweiten Positionen befinden, und in einem geringeren Ausmaß komprimiert ist, wenn sich die Arme an ihren ersten oder zweiten Positionen befinden, wodurch die Arme in ihre ersten und zweiten Positionen vorgespannt werden.

6. Eine ausfallsichere Halterung nach Anspruch 4 oder 5, bei der der Kolben (38) auf einer Spindel (36) beweglich ist.

7. Eine ausfallsichere Halterung nach einem der Ansprüche 1 bis 6, bei der der komplementäre Teil, der mit den federbelasteten Greifbacken (40) in Eingriff steht, einen Flansch (12) umfasst.

8. Eine ausfallsichere Halterung nach Anspruch 7, bei der der Flansch (12) auf der ersten Komponente (2) vorhanden ist, und die federbelastete Greifbacken (40) auf der zweiten Komponente (3) vorhanden sind.

9. Eine ausfallsichere Halterung nach einem der vorhergehenden Ansprüche, bei der die Federbelastung für die Greifbacken (40) durch eine Lastzellen-Einheit (54) unterstützt ist, die eine voreingestellte Kraft auf den Biegekraft-AuslöseMechanismus ausüben kann.

10. Eine ausfallsichere Halterung nach einem der vorhergehenden Ansprüche, bei der ein mechanischer Rücksetz-Mechanismus vorgesehen ist, um die Greifbacken (40) zwischen ihren ersten und zweiten Positionen zu bewegen.

11. Eine ausfallsichere Halterung nach den Ansprüche 4 und 10, bei der der mechanische Rücksetz-Mechanismus einen Hebel (51) umfasst, der auf den Kolben (38) einwirkt, um diesen zwischen seinen ersten und zweiten Positionen zu bewegen.

12. Eine ausfallsichere Halterung nach einem der vorhergehenden Ansprüche, bei der der Sicherheits-Auslösemechanismus weiterhin einen ausfallsicheren Torsions-Mechanismus aufweist, der auf einen Torsionskraft-Überlastzustand anspricht, um eine relative Drehbewegung der zweiten Komponente gegenüber der ersten Komponente zu ermöglichen.

13. Eine ausfallsichere Halterung nach einem der vorhergehenden Ansprüche, bei der der Sicherheits-Auslöse-Mechanismus weiterhin eine Torsionskraft-Kupplung umfasst, die auf einen Torsionskraft-Überlast-Zustand anspricht, um eine relative Drehbewegung der zweiten Komponente gegenüber der ersten Komponente zu ermöglichen.

14. Eine ausfallsichere Halterung nach Anspruch 13, bei der die Torsionskraft-Kupplung eine Kupplungsplatte (22) umfasst, die auf einen Flansch (12) einwirkt, um eine relative Drehbewegung zwischen den ersten und zweiten Komponenten zu beschränken.

15. Eine ausfallsichere Halterung nach Anspruch 14, bei der die Greifbacken (40) auf die Kupplungsplatte (22) einwirken, um sie gegen den Flansch (12) zu drücken.

16. Eine ausfallsichere Halterung nach einem der vorhergehenden Ansprüche, bei der ein Glied des Patienten einen an einem Knochen verankerten Befestigungspfosten mit einer externen Befestigungsstruktur (1) aufweist.

17. Ausfallsichere Halterung nach Anspruch 16, bei der die erste Komponente zur Verbindung mit der Befestigungsstruktur ausgebildet ist, und die ausfallsichere Halterung weiterhin einen Sicherheits-Auslöse-Mechanismus einschließt, der die Prothese normalerweise in einer starren im Wesentlichen bewegungsfreien Verbindungsbeziehung mit der Befestigungsstruktur hält.

## Revendications

1. Fixation à sécurité intrinsèque pour coupler une prothèse exosquelettique à un patient :
un premier composant (2) adapté pour le raccordement à la structure du patient ; et
un second composant (3) adapté pour le raccordement à la prothèse ;
la fixation à sécurité intrinsèque comprenant en outre un mécanisme de déblocage de sécurité retenant normalement la prothèse dans une relation interconnectée sensiblement dépourvue de mouvement par rapport au patient, ledit mécanisme de déblocage de sécurité répondant à une condition de surcharge de force pour permettre le mouvement relatif entre la prothèse et le patient ;
ledit mécanisme de déblocage de sécurité comprenant un mécanisme de déblocage de force de flexion sensible à une condition de surcharge de force de flexion pour permettre la séparation dudit second composant par rapport audit premier composant, le mécanisme de déblocage de force de flexion comprenant une pluralité de mâchoires à ressort (40) positionnées sur un premier composant parmi les premier et second composants, et étant adaptées pour mettre en prise une partie complémentaire d'un second composant parmi les premier et second composants,
**caractérisée en ce que** les mâchoires (40) peuvent être pivotées dans une configuration de rappel réversible entre une première position dans laquelle elles mettent en prise la partie complémentaire pour fixer les premier et second composants l'un sur l'autre, et une seconde position dans laquelle les premier et second composants peuvent se séparer.

2. Fixation à sécurité intrinsèque selon la revendication 1, dans laquelle les mâchoires à ressort (40) comprennent chacune un bras s'étendant radialement (39) ayant une extrémité interne et une extrémité externe, l'extrémité externe de chaque bras portant une partie de doigt (53).

3. Fixation à sécurité intrinsèque selon la revendication 2, dans laquelle la partie de doigt (53) de chaque bras s'étend d'une manière rétrograde par rapport au bras (39).

4. Fixation à sécurité intrinsèque selon la revendication 2 ou la revendication 3, dans laquelle l'extrémité interne de chaque bras est fixée à un piston (38) mobile entre une première position dans laquelle l'extrémité interne de chaque bras est inférieure à l'extrémité externe, et une seconde position dans laquelle l'extrémité interne de chaque bras est plus grande que l'extrémité externe.

5. Fixation à sécurité intrinsèque selon la revendication 4, dans laquelle chaque bras est prévu avec un ressort de compression (41), de sorte que le ressort de compression est comprimé sur une plus grande étendue lorsque les bras sont dans n'importe quelle position entre leurs première et seconde positions, et sur une moindre étendue lorsque les bras sont dans leur première ou seconde position, afin de solliciter ainsi les bras dans leurs première et seconde positions.

6. Fixation à sécurité intrinsèque selon la revendication 4 ou la revendication 5, dans laquelle le piston (38) est mobile sur une broche (36).

7. Fixation à sécurité intrinsèque selon l'une quelconque des revendications 1 à 6, dans laquelle la partie complémentaire mise en prise par les mâchoires à ressort (40) comprend une bride (12).

8. Fixation à sécurité intrinsèque selon la revendication 7, dans laquelle la bride (12) est présente sur le premier composant (2) et les mâchoires à ressort (40) sont présentes sur le second composant (3).

9. Fixation à sécurité intrinsèque selon l'une quelconque des revendications précédentes, dans laquelle le chargement par ressort pour les mâchoires (40) est assisté par une unité de cellule de charge (54) capable d'exercer une force prédéterminée sur le mécanisme de déblocage de force de flexion.

10. Fixation à sécurité intrinsèque selon l'une quelconque des revendications précédentes, dans laquelle un mécanisme de réinitialisation mécanique est prévu pour déplacer les mâchoires (40) entre leurs première et seconde positions.

11. Fixation à sécurité intrinsèque selon les revendications 4 et 10, dans laquelle le mécanisme de réinitialisation mécanique comprend un levier (51) agissant sur le piston (38) pour le déplacer entre ses première et seconde positions.

12. Fixation à sécurité intrinsèque selon l'une quelconque des revendications précédentes, dans laquelle le mécanisme de déblocage de sécurité comprend en outre une mécanisme de torsion à sécurité intrinsèque sensible à une condition de surcharge de force de torsion pour permettre le déplacement en rotation relatif dudit second composant par rapport audit premier composant.

13. Fixation à sécurité intrinsèque selon l'une quelconque des revendications précédentes, dans laquelle le mécanisme de déblocage de sécurité comprend en outre un embrayage de force de torsion sensible à une condition de surcharge de force de torsion pour permettre le déplacement en rotation relatif dudit second composant par rapport audit premier composant.

14. Fixation à sécurité intrinsèque selon la revendication 13, dans laquelle l'embrayage de force de torsion comprend un disque d'embrayage (22) agissant sur une bride (12) pour limiter le mouvement de rotation relatif entre les premier et second composants.

15. Fixation à sécurité intrinsèque selon la revendication 14, dans laquelle les mâchoires (40) agissent sur le disque d'embrayage (22) pour le pousser contre la bride (12).

16. Fixation à sécurité intrinsèque selon l'une quelconque des revendications précédentes, dans laquelle un membre du patient a un montant de montage d'os ancré avec une structure de fixation externe (1).

17. Fixation à sécurité intrinsèque selon la revendication 16, dans laquelle le premier composant est adapté pour le raccordement à la structure de fixation et la fixation à sécurité intrinsèque comprend en outre un mécanisme de déblocage de sécurité retenant normalement la prothèse dans une relation interconnectée rigide sensiblement dépourvue de mouvement par rapport à la structure de fixation.
